Europäisches Patentamt

European Patent Office

Office européen des brevets

(11) Publication number: **0 232 185**
**A2**

## (12) EUROPEAN PATENT APPLICATION

(21) Application number: **87301070.6**

(22) Date of filing: **06.02.87**

(51) Int. Cl.⁴: **A 61 B 5/02**

(30) Priority: **07.02.86 JP 26487/86**
**30.04.86 JP 101781/86**

(43) Date of publication of application:
**12.08.87 Bulletin 87/33**

(84) Designated Contracting States: **DE FR GB IT**

(71) Applicant: **Takahashi, Masakatsu**
**4-6, Narihira-cho**
**Ashiya-shi Hyogo-ken (JP)**

(72) Inventor: **Takahashi, Masakatsu**
**4-6, Narihira-cho**
**Ashiya-shi Hyogo-ken (JP)**

(74) Representative: **Hillier, Peter et al**
**Reginald W. Barker & Co., 13, Charterhouse Square**
**London, EC1M 6BA (GB)**

(54) A blood pressure measuring device.

(57) A blood pressure measuring device has a probe (1) having
a blood flow sound detector (3) at the bottom thereof to be
pushed to a skin over an artery examined and being a hollow
body in a size sufficient to cover at least width of the artery at
the position to be contacted therewith which changes the form
thereof elastically. The probe (1) is airtightly and removably
connected by a connecting member (5) to a pressure gauge (2)
which indicates the changes in pressure. The connecting
member (5) has enough elasticity to restore to the original
shape thereof.

FIG.1

## Description

### A BLOOD PRESSURE MEASURING DEVICE

#### FIELD OF THE INVENTION AND RELATED ART STATEMENT

This invention relates to a blood pressure measuring device used in hospitals and ordinary homes to diagnose a health condition of human being.

It is necessary to measure blood pressure regularly or according to the physical condition in order to grasp the changes in blood pressure and diagnose the health condition. If blood pressure however must be taken at a hospital every time, this makes the burden heavy timewise and economically for not only patients but also healthy persons. On the other hand, as blood pressure can be measured simply in comparison even by an amateur, blood pressure measuring devices which can be handled simply in ordinary homes have been developed and put on the market in these days.

However, this type of blood pressure measuring device for home use disclosed at present is used, like those which have been used in hospitals, by winding a rubber air bag called a manschette round an upper arm of human being and applying a blood flow sound detector to a brachial artery.

The problem with the above-mentioned blood pressure measuring device is that it is very troublesome to wind the manschette round the upper arm and that it is difficult to position the blood flow sound detector provided inside the manschette on the elbow joint artery exactly and thus it is difficult to make an exact measurement of blood pressure. Furthermore, as the manschette is wound round the upper arm, the resistance pressure of a partial tissue is added according to a rate of corpulence of a person examined and for this reason, there exists the problems that exact measurement of blood pressure can not be made.

Under these circumstances, the applicant designed a blood pressure measuring device disclosed in the Japanese Published Examined Utility Model Application No. 12963/1986.

In this blood pressure measuring device, a pressure sensitive device is fitted to a blood flow detecting device. However, when elasticity of a fitting part of the pressure sensitive device deteriorated for some reason, there is trouble that the pressure sensitive device happens to come off and that pressure is not be able to indicate exactly with air leak.

#### SUMMARY OF THE INVENTION

This invention relates to a blood pressure measuring device which comprises a pressure sensitive means which has a blood flow sound detector at the bottom thereof to be pushed to a skin over an artery examined and is able to be transformed elastically, a pressure indicating means which is connected airtightly to the pressure sensitive means and indicates the changes in pressure when the pressure sensitive means is pressed and a blood flow informing means which informs the existence of a blood flow by a signal from the blood flow sound detector;

the pressure sensitive means being in the form of a hollow body in a size sufficient to cover at least a width of an artery at the position to be contacted therewith and having an opening connected to the pressure indicating means,

The pressure indicating means having an air flow pipe which forms a passage of pressured air from the pressure sensitive means and

the pressure sensitive means and the pressure indicating means being airtightly connected by a connecting member which is fitted to the circumference of the opening of the pressure sensitive means and removably engaged on the air flow pipe of the pressure indicating means and has enough elasticity that can return to its original shape.

#### BRIEF DESCRIPTION OF THE DRAWINGS

Fig. 1 is a front view of a blood pressure measuring device embodying the present invention;

Fig. 2 is a vertical sectional view of the pressure sensitive means;

Fig. 3 is a front view of the embodiment in which the pressure sensitive means is pressed;

Fig. 4 is a block diagram of the blood flow informing means of the embodiment;

Fig. 5 shows the pressure sensitive means with the connecting member removed from the pressure indicating means;

Fig. 6 shows another embodiment and corresponds to Fig. 5;

Fig. 7(a), (b) and (c) are diagrams showing the movements of a blood pressure measuring device of one more different embodiment of the present invention fitted to a bracelet; and

Fig. 8(a), (b) and (c) are diagrams showing the movements of the valve provided inside the air flow pipe.

#### DETAILED DESCRIPTION OF THE PREFERRED EMBODIMENTS

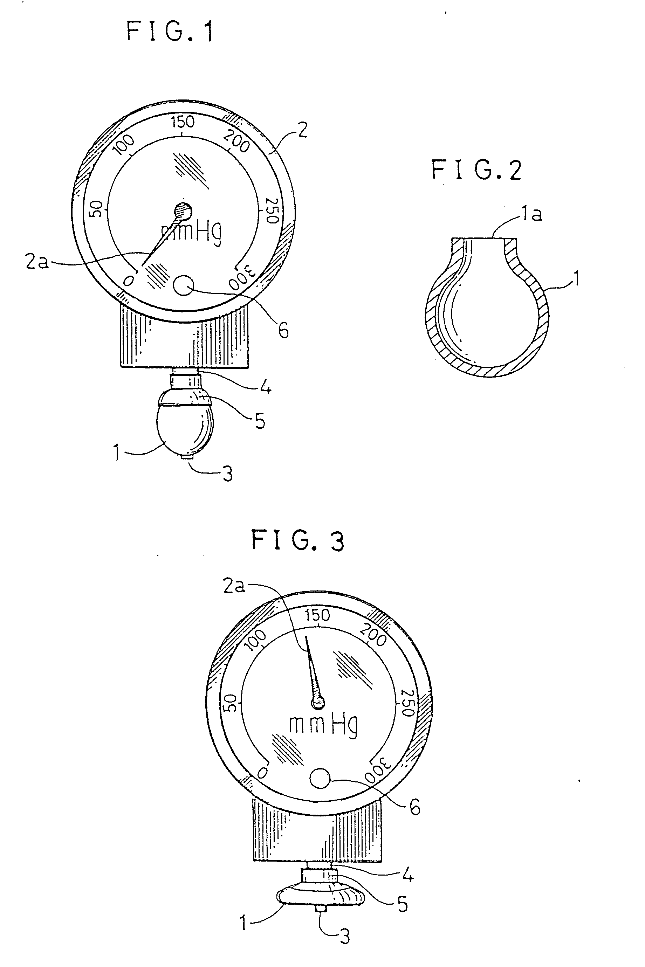

In Fig. 1 showing a blood pressure measuring device (Tycos-type) of a embodiment of this invention, 1 is a probe which is a pressure sensitive means in which a soft rubber film is used as an elastic material which can be restored to a original shape, and the whole of the probe forms a hollow body, i.e. a hollow globular body a little thicker or larger than the thickness or width of the inspected artery (for example, about 10 mm in diameter for testing the radius artery) and has, at its one end, an opening 1a which is connected airtightly under normal pressure to a pressure receiving part of a pressure indicating means (pressure gauge) 2. (Fig. 2 shows a vertical sectional view of the probe 1.)

The air pressure in the hollow inside of the probe 1 is kept at 0 mmHg under a normal condition, that is, no pressure is applied. The probe 1 has enough elasticity to withstand a maximum air pressure of 300 mmHg, namely when pressure given to the inside reaches the maximum, and to let the inside air pressure return to the original 0 mmHg automatically when the compression is taken off. The probe 1 may be a semicircular globular shape though not shown in the drawing. Moreover, probes in a variety of sizes may be exchangeably used. For example, the probe in a small size is used for children and one in a large size for adults. The size when the probe is in original state, is one which can cover at least the width or thickness of an artery at the position to be contacted with the pressure sensitive means, specifically about 1 - 30 mm in diameter, preferably about 10 mm.

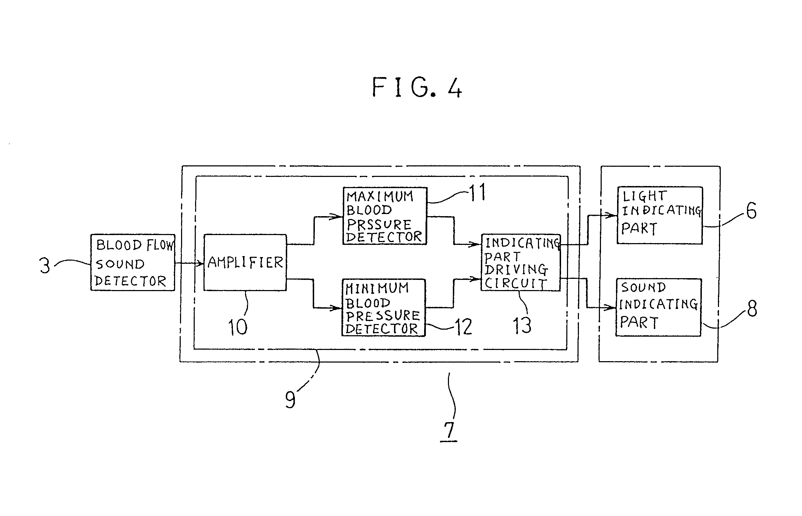

Installed at the bottom of the probe 1, which is pushed against a skin over the artery examined, is a blood flow sound detector 3. For the blood flow sound detector 3, a microphone about the size of a grain of rice is appropriate. A pointer 2a of the pressure indicating means 2 indicates 0 under normal pressure and moves as the probe 1 changes its shape as shown in Fig. 3. 4 is an air flow pipe projecting downward from the bottom of the pressure indicating means 2. 5 is a connecting member which has elasticity to return to its original shape and connects the probe 1 to the pressure indicating means 2 airtightly. 6 is a light indicating part of a blood flow informing means 7 and is composed of a light emitting diode for example. The blood flow informing means 7 is provided inside of the pressure indicating means 2 and, as shown in Fig. 4, it is composed of the light indicating part 6, a sound indicating part 8 which emits an electronic sound with an oscillating element and an indicating circuit 9 which drives the light indicating part 6 and the sound indicating part 8. In the indicating circuit 9, an electric signal which is output from the blood flow sound detector 3 is amplified by an amplifier 10 and an indicating part driving circuit 13 is worked by output signals from a maximum blood pressure detector 11 and a minimum blood pressure detector 12 which work by the amplified electric signal.

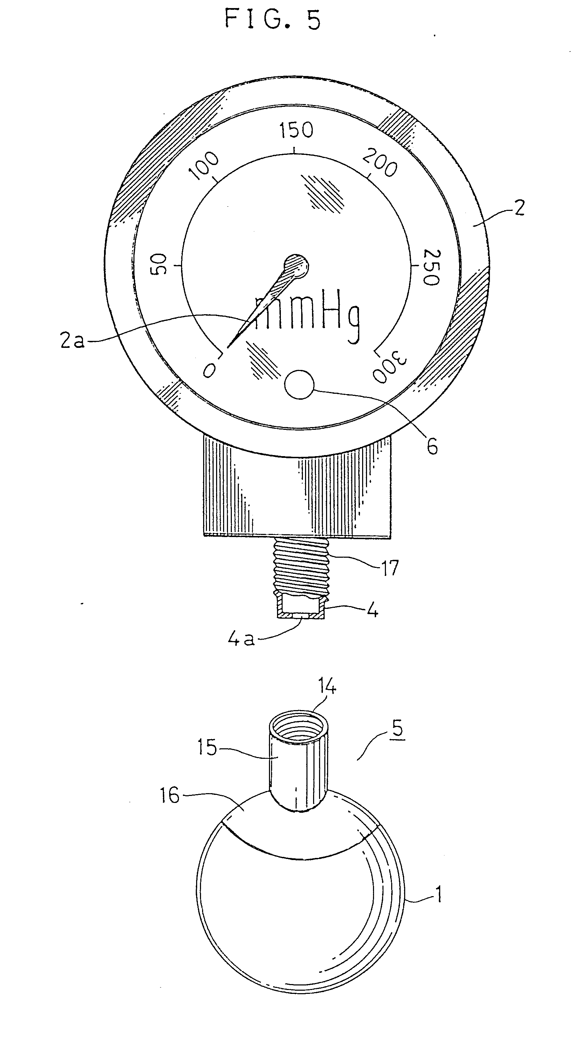

Now referring to Fig. 5, how the probe 1 is fitted to the connecting member 5, that is, a fitting structure will be explained.

The connecting member 5 is composed of a connecting pipe 15 and a connecting flange 16; the flange 16 extends in a unit from the end fringe of the connecting pipe 15. The inner circumference of the connecting pipe 15 is tapped spirally to form a part inserted 14. That is, a part inserted 14 has screw threads. And the connecting flange 16 of the connecting member 5 is stuck on the circumference of the opening 1a of the probe 1 with adhesives so that the probe 1 is held together with said connecting member 5 as a unit.

On the other hand, the outer circumference of the air flow pipe 4 projecting from the pressure indicating means 2 is died spirally to form an inserting part 17. That is, an inserting part 17 has screw threads. Therefore, when the probe 1 is fitted to the pressure indicating means 2, the connecting pipe 15 of the connecting member 5 is screwed on the air flow pipe 4 of the pressure indicating means 2. By putting pressure upon the probe 1 fitted in such a way to change its shape. pressured air is led to the pressure indicating means 2 through the opening 1a and a gas passage 4a and thus the pressure is indicated.

Blood pressure is measured by being contacted the probe 1 at the position over a blood vessel (the radius artery examined and compressing the probe 1. The maximum and the minimum blood pressure can be known by closing the blood vessel once with this compression and then lessening the compression gradually, and when the blood flow sound detector 3 is detecting the blood flow sound at the time of blood pressure between its maximum and minimum, the indicating circuit 9 drives the sound indicating part 8 and the light indicating part 6 and consequently the blood flow informing means 7 works and informs a user that there is a blood flow.

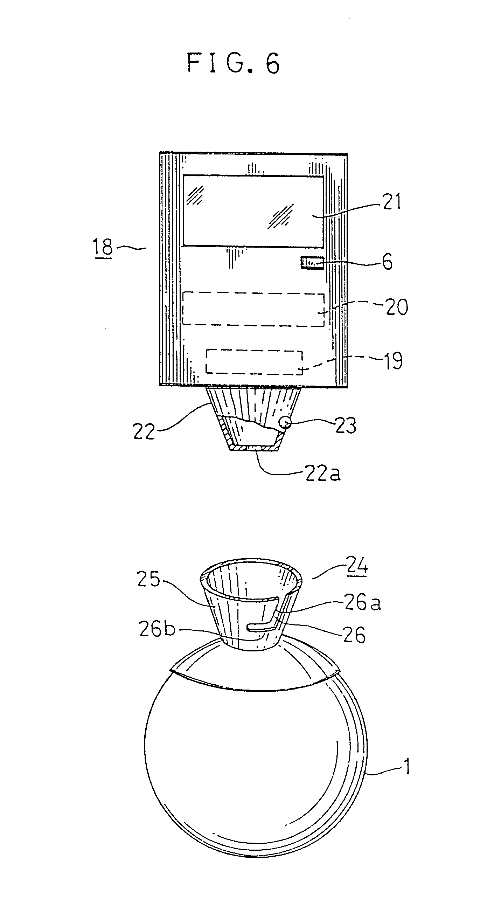

The blood pressure measuring device shown in Fig. 6 is another embodiment of this invention. A pressure indicating means 18 is composed so that the pressure of introduced gas is transformed electronically and indicated with a column of mercury by the use of piezoelectric elements and up to 300 mmHg can be measured. The pressure indicating means 18 has a pressure measuring part 19, an electron volume changing part 20 and pressure indicating part 21 built-in. Although the indicating method of the pressure indicating part 21 is digital display, also analogue display with a pointer suffices.

An air flow pipe 22 or another embodiment mentioned above has an external form which tapers downward from the bottom of the pressure indicating means 18, namely a truncated cone shape, and a convex part (dowel) which is an inserting part 23 is provided at a suitable place on the outer circumference of the air flow pipe 22. A gas passage 22a is provided at the bottom of the air flow pipe 22 in the same way as the embodiment of this invention. A connecting member 24 has an inverse truncated cone shape corresponding to the shape of the air flow pipe 22 and has an L-shaped slit cut from the rim of a connecting pipe 25 to form a part inserted 26 which receives the inserting part 23. Therefore, to fit the probe 1 to the pressure indicating means 18, first the connecting pipe 25 is engaged on the air flow pipe 22 by setting the inserting part 23 to a vertical slit 26a of the part inserted 26 and then the inserting part 23 is engaged in a horizontal slit 26b by turning the connecting member 24.

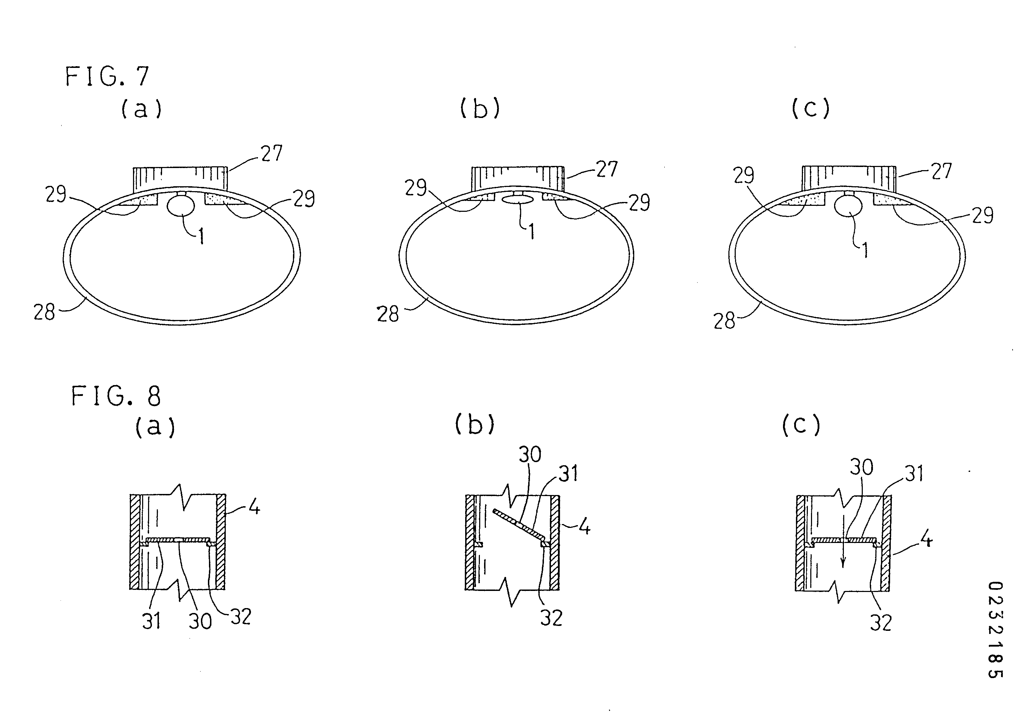

The device shown in Fig. 7 is one more different embodiment of this invention. A pressure indicating means 27 is of an electronic type in the same way as another embodiment and has said probe 1 on the rear of the pressure indicating part. The pressure indicating means 27 is fitted to a bracelet 28. Provided on the right and left side of the probe 1 are elastic materials 29 to assist the restoring force of the probe 1 and the elastic materials 29 are fitted to the inner circumference of the bracelet 28.

Fig. 7(a) shows the probe 1 without load. Fig. 7(b) shows that a blood vessel pressed by the probe 1 is close. At this time, the elastic materials 29 are pressed and transformed together with the probe 1. Fig. 7(c) shows that the pressure is weakened gradually. With the restoring force of the elastic materials 29 at this time, the probe 1 can be restored smoothly.

Moreover, the form and material of the elastic materials 29 are not limited specially if they have the above-mentioned effects.

Fig. 8 shows a sectional view of a check valve 31 with a small hole 30 (to prevent the inverse flow of the probe 1) provided in the air flow pipe 4 which is so designed that air returns gradually through the small hole 30 to the inside of the probe 1. Therefore, the indicating valve of a pressure indicating means changes gradually and the maximum and the minimum blood pressure can be read exactly. 32 in Fig. 8 is a valve seat which rotatably fixes the check valve 31 to open and close freely. For the valve seat 32 and the check valve 31, a magnet is formed in a unit and when the check valve 31 is close, it is attracted by the magnet.

Fig. 8(a) shows the probe 1 without load. Fig. 8 (b) shows that the check valve 31 is open when a blood vessel pressed by the probe 1 is close and that air flows in the direction of the pressure indicating means. Fig. 8(c) shows that the pressure is weakened gradually. At this time the check valve 31 i close and air returns gradually through the small hole 30 to the inside of the probe 1.

As is clear from the above embodiments, the blood pressure measuring device of the present invention provides various merits as follows.

As the pressure sensitive means is a hollow body in such extent size that a thickness of the artery is covered, the changes in compressed pressure inside the pressure sensitive means can be transferred to the pressure indicating means exactly and sensitively, and at the same time, the pressure sensitive means can be applied to and compressed in a right position over a blood vessel.

Besides that, as the pressure sensitive means is also removably connected with the connecting member, it can be easily changed into another pressure sensitive means which has different size to be selected by the width of the artery contacted.

In addition, blood pressure can be measured simply only by applying the pressure sensitive means, which is formed by elastic material having elasticity, to the side wall of a blood vessel and by working the pressure sensitive means, and no manschette (rubber globe belt wound round the upper arm) is required. Therefore, blood pressure in blood vessels at any part of the body can be measured with a compact device and everybody can take blood pressure simply. Furthermore, as the compression of blood vessels and the measurement of a blood flow sound can be made in a narrow range, blood pressure near a wrist can also be taken. Therefore, compared with the case where only blood pressure near the upper arm can be measured, there is no such a primary factor that blood vessels are compressed by a rolled up sleeve of clothes and thus blood pressure can always be measured exactly.

## Claims

1. A blood pressure measuring device which comprises a pressure sensitive means which has a blood flow sound detector at the bottom thereof to be pushed to a skin over an artery examined and is able to be transformed elastically, a pressure indicating means which is connected airtightly to the pressure sensitive means and indicates the changes in pressure when the pressure sensitive means is pressed and a blood flow informing means which informs the existence of a blood flow by a signal from the blood flow sound detector;
the pressure sensitive means being in the form of a hollow body in a size sufficient to cover at least a width of an artery at the position to be contacted therewith and having an opening connected to the pressure indicating means,
the pressure indicating means having an air flow pipe which forms a passage of pressured air from the pressure sensitive means and
the pressure sensitive means and the pressure indicating means being airtightly connected by a connecting member which is fitted to the circumference of the opening of the pressure sensitive means and removably engaged on the air flow pipe of the pressure indicating means and has enough elasticity that can return to its original shape.

2. A blood pressure measuring device according to claim 1 wherein the hollow body has an outer diameter of 1 -30 mm.

3. A blood pressure measuring device according to claim 1 wherein the hollow body has an outer diameter of about 10 mm.

4. A blood pressure measuring device according to claim 1 wherein the hollow body is a hollow globular body.

5. A blood pressure measuring device according to claim 1 wherein the air flow pipe has an inserting part at the outer circumference thereof.

6. A blood pressure measuring device according to claim 1 wherein the connecting member is composed of a connecting pipe having a part inserted which is put in an inserting part of the air flow pipe and a connecting flange extending in a unit from the end fringe of the connecting pipe.

7. A blood pressure measuring device according to claim 5 wherein the inserting part has screw threads.

8. A blood pressure measuring device according to claim 6 wherein both of the inserting part inserted have screw threads.

9. A blood pressure measuring device according to claim 6 wherein the connecting pipe is of an inverse truncated cone shape and the air flow pipe is of a truncated cone shape having an external form which approximately equals the internal form of the connecting pipe.

10. A blood pressure measuring device according to claim 9 wherein the inserting part is a convex provided on the outer circumference of the air flow pipe and the part inserted is an L-shaped slit provided in the connecting pipe.

F I G.1

F I G.2

F I G. 3

FIG. 4

0232185

FIG. 5

0232185

FIG. 6

FIG. 7

(a)

(b)

(c)

FIG. 8

(a)

(b)

(c)